Europäisches Patentamt

(19) European Patent Office

Office européen des brevets

(11) Publication number: **0 112 563**
A2

(12) # EUROPEAN PATENT APPLICATION

(21) Application number: **83113007.5**

(22) Date of filing: **22.12.83**

(51) Int. Cl.³: **A 61 K 37/02, A 61 K 35/16**

(30) Priority: **27.12.82 US 453073**

(43) Date of publication of application: **04.07.84**
**Bulletin 84/27**

(84) Designated Contracting States: **AT BE CH DE FR GB IT LI LU NL SE**

(71) Applicant: **USV PHARMACEUTICAL CORPORATION, 1 Scarsdale Road, Tuckahoe New York (US)**

(72) Inventor: **Landaburu, Ricardo H., 1 Magnolia Drive, Rye Town New York 10573 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath, Maximilianstrasse 58, D-8000 München 22 (DE)**

(54) Solvent treatment of plasma protein products.

(57) Plasma protein products are slurried in the dry state with an organic solvent to remove virus infectivity.

# SOLVENT TREATMENT OF PLASMA PROTEIN PRODUCTS

The present invention relates to rendering biological products, and particularly plasma fractions, free of transmissible infectious hepatitis viruses, and in particular Hepatitis B virus ("HBV") and hepatitis non-A-non-B-virus(es) ("HnAnBV"), free of pyrogens, and free of thrombogenic (i.e. thrombosis-inducing) materials.

Continuing concern for the safety of recipients of biological protein products has inevitably focused on the potential transmission of serum hepatitis during blood component therapy, which may be caused by the presence of even undetectable levels of infective viruses in the donor plasma pool. Though tested in each case by state of the art methods, it is widely understood that low but infectious levels of HBV may still occur undetectable in plasma, and no in vitro test has been devised which predicts the presence or absence of the HnAnB virus(es).

While the development of screening tests for hepatitis B has been of limited value in reducing transmission of the disease, the identification of this virus (as well as the hepatitis A virus) has led to the recognition of a third virus or, more accurately, a class of virus(es), referred to in the art as hepatitis non-A-non-B virus(es), which is apparently responsible for the majority of cases of hepatitis transmitted by blood plasma derivatives. Tests for HnAnBV are not yet commercially available for widespread screening. Thus, it would be desirable to be able to reduce hepatitis infectivity in biological products without losing biological activity.

Pyrogens are lipopolysaccharides (LPS) derived from the outer cell wall of gram-negative bacteria. They are toxic materials which are also known as endotoxins to distinguish them from toxic substances synthesized and excreted by the intact bacterium. Pyrogens have numerous biologic activities which include the production of fever, activation of clotting mechanisms and induction of shock. Consequently, it is essential that pyrogenic substances be removed and that the causative bacteria be rendered innocous by sterilization or other such treatment of the final plasma product.

Thrombosis, i.e., the undesirable induction of clotting in blood, is a property of certain plasma protein products such as Factor IX Complex. Thrombosis-inducing potential in this preparation is believed to be associated with partial activation of the Coagulation Factor or with the presence of lipids (probably of platelet origin) in the preparation.

U.S. Patent No. 3,682,835 discloses that blood serum or plasma from which sodium, potassium and calcium cations have been extracted and which has about the pH of the starting material can be lyophilized, treated with an organic solvent to extract lipoproteins, and then reconstituted with water to produce a stabilized analytical control standard. This patent does not describe or suggest the preparation of a product for therapeutic administration, and does not relate to the preservation of the biological activity of the material under treatment. The patent also does not suggest any method for reducing viral infectivity and thrombogenicity of a plasma product.

The present invention comprises a process for preventing the transmission of virus having structural lipids by an infectable plasma protein product, while retaining at least about 25% of the biological activity of the plasma protein product, comprising

-3-

(a) forming a slurry by mixing the plasma protein product in the dry state with a solvent which is capable of dissolving lipids and in which the plasma protein product is essentially insoluble,

(b) maintaining the slurry under conditions effective to extract virus into the organic solvent while lowering the biological activity of said product by no more than about 75%, and then

(c) removing the solvent in the liquid state from the plasma protein product, thereby providing the product in a dry, purified state.

This product can be formulated into an injectable composition by dissolution in a pharmaceutically-acceptable liquid carrier.

The present invention is applicable to all types of plasma protein products for therapeutic use. Examples of such products are plasma and plasma fractions such as antihemophilic factor A (AHF, Factor VIII); Factor IX; Factor IX complex (Factors II, VII, IX and X); Antithrombin III; fibronectin; fibrinogen; gamma globulin; albumin; placenta plasma proteins; and the like. The plasma protein is frequently obtained by Cohn fractionation of pooled plasma, which as is recognized in this art carries a risk of possessing hepatitis and other virus infectivity.

The present invention is particularly directed to rendering such biological material non-infectious as to hepatitis viruses, and in particular as to HBV, HB Delta Factor and HnAnBV. It is applicable more generally to removal of any viruses having structural lipids, such as cytomegalovirus, Epstein-Barr virus, Hepadnavirus, Vaccinia, and the like. Indeed, the invention is also applicable to the removal of lipids; complex molecules having one or more lipids in their structure; and multimolecular

-4-

complexes comprised wholly or partially of lipids. Examples of these are lipoproteins, pyrogens and other lipopolysaccharides, fatty materials such as caprylate, and platelet lipoproteins and the like thrombogenic material.

A significant feature of the present invention is that the plasma protein product is in the dry state when it is contacted with an organic solvent. The invention is therefore particularly advantageous for treatment of plasma protein products which are susceptible to precipitation from aqueous solutions thereof, and to resultant loss of activity and denaturation, when the aqueous solutions are treated with organic solvents. When the protein precipitates, it loses its biological activity and it cannot readily be redissolved. The products to which this invention is directed can conveniently be defined as any plasma protein product more than about 20% of which, and even more than about 10% of which is precipitated from a 0.1 wt. % aqueous solution thereof at 10°C by mixing such aqueous solution with 20 vol. % of chloroform. Products which are particularly susceptible to precipitation from aqueous solution by organic solvents, and to which the present invention is particularly directed, include Factor VIII, Factor IX complex, albumin, and Antithrombin III. By contrast, these and all other plasma protein products treated in accordance with the present invention can be easily and completely redissolved into aqueous solutions for therapeutic administration thereof with little or no loss of biological properties. The invention is described below with particular reference to commercial Factor VIII, which as is recognized in this art is available as an aqueous solution containing plasma protein components and having a small statistical probability of possessing hepatitis infectivity.

In practice, the plasma protein product is treated in the dry state, preferably as finely divided powder. Some products may be available as dry powders, in which case no further drying treatment may be necessary. In some cases, the product is in the form of an aqueous solution, in which case a drying treatment must be employed. The drying can be carried out by conventional freeze-drying or lyophilization methods, in which the solution is dried from the frozen state under a high vacuum (e.g., 10 to 200 microns) at a temperature of typically -40°C to 40°C for e.g. 24 to 96 hours such that the water component sublimes. Residual moisture of about 0.1 to about 0.2 wt. % can be tolerated.

The dry material is then slurried in an organic solvent which is a liquid at 25°C and atmospheric pressure, which is capable of dissolving lipids, and in which the plasma protein product is essentially insoluble. Suitable solvents include benzene; furans, alkanols, ketones, ethers, alkyl-substituted benzene, alkanes, and cyclo-alkanes, any of which may have up to 8 carbon atoms; and methane or ethane having 2 or more halogen substituents selected from the group consisting of fluorine and chlorine; and mono- and biphasic mixtures thereof. Examples of preferred solvents are chloroform, acetone, diethyl ether, benzene, toluene, xylene, methanol, ethanol, isopropanol, n-octanol, tetrahydrofuran, and mono- and biphasic mixtures thereof. The amount of solvent should constitute about 2 to about 20 ml per gram of solid material, and preferably about 5 to 10 ml/gram.

Larger amounts of solvent can be added, but do not add appreciably to the extraction efficiency. The slurry is maintained, preferably with gentle agitation to increase solid-liquid contact, for a period of time effective to extract the virus into the organic solvent. Some extraction occurs almost immediately, and virus continues to be extracted over a period of up to about 8 hours, and preferably up to about 6 hours. The slurry should be maintained at least about 5 to 10 minutes, and preferably at least about 1 hour. Continuing the extraction too long ensures extraction and destruction of virus present in even heavily virus-infested plasma protein product but also risks adverse reaction between the solvent and the plasma protein product. The solvent should be removed from the slurry before the plasma protein product suffers excessive deactivation or denaturation, and suitable extraction times to avoid this result can readily be determined by measurements of the protein's biological activity following treatments.

While the extraction is satisfactory when carried out at room temperature, too high a temperature damages the plasma protein; extraction at below about 30°C is preferred, temperatures of about 0°C to 20°C are more preferred, and more preferred still are temperatures of about 2°C to about 10°C.

At least a portion of the virus is still viable when initially extracted into the organic solvent, so that the mechanism by which the transmission of virus by the product is prevented in this invention is a combination of extraction of viable virus and destruction of virus by the solvent. Continued contact with the organic solvent, either before or following extraction into the solvent or following removal of the virus-bearing solvent from the plasma product eventually deactivates or destroys the virus. It is believed that the mechanism of the present invention, involving at least partial extraction of viable virus, has not previously been recognized.

Thus, in another embodiment of this invention the dry plasma material and the solvent are slurried together long enough to extract viable virus into the solvent, and the solvent phase is removed from the slurry while some and even up to about 25% or 50% of the extracted virus is still viable. That is, contact between the solvent and the plasma protein can be discontinued before all virus in the slurry is destroyed. This embodiment provides all the other advantages discussed herein and provides two additional advantages. One is that shortening the period of time in which the solvent and the plasma protein product remain in contact further protects the plasma protein product from loss of activity, denaturation, or other damage from the solvent. Another advantage is that the total time needed to treat a quantity of plasma product is shortened by the length of time that the plasma product would be held in the slurry while the solvent destroys all the virus including that which has already been extracted. Each extraction stage can last for about 5 minutes to about 60 minutes, or longer, and extractions of viable virus can be repeated to progressively lower the viral activity in the plasma product.

While the present invention contemplates solvent treatment in which at least about 25% of the biological activity is retained, in the more preferred embodiments over about 50% of the activity, and even more preferably at least about 75% of the biological activity is retained. In the most preferred embodiments, the plasma product retains over about 90% of its biological activity, and in the best mode of the invention essentially no activity is lost.

Besides providing high biological activity in the recovered plasma product, the present invention permits recovery of essentially all of this product; that is, there is little or no dissolution of biologically active plasma protein material. Another advantage of the process of this invention is that when the biological product retains 90% or more of its activity it also undergoes essentially no denaturation. This is an important advantage because material that has been partially denatured can still possess biological activity, but can cause other side reactions in some patients to whom the material is administered.

At the end of the slurrying/extraction step, the solvent is removed from the slurry. The solvent must be kept in the liquid state during the extraction and when it is separated from the plasma protein product; evaporation, while inviting because of the low vapor pressure of the typical solvent, is not acceptable because of the likelihood that infectious viral material, which term is hereby defined to include any combination of deactivated viruses, virus fragments, and/or viable virus, would be left behind in the plasma protein product. Satisfactory techniques for separating the solvent from the plasma product include decanting, filtration, and a combination thereof. The solvent, after removal from the the plasma protein product, can be recovered for reuse by e.g. distillation.

The product remaining after removal of the solvent is dry and purified, and can readily be reconstituted into a therapeutically administrable product by e.g. dissolving the amount to be administered in a therapeutically-acceptable liquid carrier.

Among other advantages of the present invention besides those mentioned above, it is noted that treating the plasma protein product in the dry state avoids dilution of the product, and thus employs the organic solvent more efficiently while reducing the overall volume of liquids that must be handled. Attempts to reduce the tendency to precipitate by reducing the concentration of the plasma protein product before adding organic solvent, serve to decrease the yield per unit of solvent added, and greatly increase the amount of solvent that must be handled. Another advantage of the present invention is that plasma protein products can be treated which cannot readily be dissolved in satisfactory concentrations into aqueous solution for treatment by techniques for treatment of protein solutions. Furthermore, the absence of an aqueous phase enhances the efficiency of the present invention because of the reduction or removal of hydrophilic components on the surface of the virus matter which would set up surface tension that would hinder the access of the solvent, particularly a water-immiscible solvent, to the surface of the virus. In addition, the process steps described herein of the present invention can be used to reduce the thrombogenicity of plasma protein products, thereby increasing the therapeutic utility of products treated by this process and especially of Factor IX and Factor IX complex.

The conditions described herein which are effective to remove virus material from the plasma protein product are also effective to remove pyrogens therefrom.

The invention is also adaptable to continuous operation, in which quantities of dry plasma protein product and organic solvent are fed continuously, at feed rates chosen as set forth herein to provide the desired slurry density, into a vessel adapted to maintain the temperature of the slurry at or below about 25°C and adapted to discharge the slurry through a filter so as to provide dry, purified plasma product and viral matter-bearing solvent. The residence time in the vessel is selected by adjusting feed rates, discharge rate, and vessel volume, to provide that solvent-protein contact is maintained long enough to destroy all virus or, preferably, long enough so that the solvent discharged from the vessel contains

-10-

some viable virus (which can subsequently be destroyed by the solvent or otherwise).

The present invention will be described in the following Examples.

-11-

EXAMPLE 1

This Example demonstrates the removal of virus activity by the process of this invention. Vaccinia virus was employed in the tests rather than hepatitis because Vaccinia can be more readily assayed. More importantly, Vaccinia is believed to be more resistant to the action of organic solvents than hepatitis viruses, so that demonstration of the effectiveness of the invention as to Vaccinia virus proves that the invention is also effective as to hepatitis virus.

A batch of Factor VIII (aqueous solution) commercially manufactured in the United States was inoculated with an amount of Vaccinia virus (ATCC No. VR 862 Lot 1) such that when the product was placed in vials (30 ml per vial) each vial contained about $10^{4.7}$ $TCID_{50}$/ml as determined by assay of an untreated vial. ($TCID_{50}$ is recognized in this art to mean the dose that infects 50% of a standardized tissue culture). The contents of each vial were then lyophilized, and the dry contents of certain vials (400 mg each) were slurried in 10 ml of chloroform at 20°C for 20 minutes or 6 hours. The chloroform was then removed from the slurry by filtration, in which case the filtrate was assayed for Vaccinia acitivity, or by evaporation, in which case the residue was assayed for Vaccinia activity.

The chloroform filtrate obtained after 20 minutes assayed $10^{1.7}$ $TCID_{50}$/ml, indicating that some viable virus had been extracted from the factor VIII into the solvent. When the chloroform was removed by evaporation after 20 minutes, the virus activity of the Factor VIII was $10^{3.7}$ $TCID_{50}$/ml, indicating signifcant reduction of virus infectivity in the Factor VIII. It is noted, though, that evaporation of the chloroform returned into the Factor VIII some viable virus which had been extracted into the chloroform.

In the two other parallel tests, wherein the chloroform was either filtered off or evaporated off after 6 hours, the filtrate and the residue following evaporation, each assayed less than $10^{1}$ $TCID_{50}$/ml, indicating substantial destruction of viral infectivity.

-13-

## EXAMPLE 2

To demonstrate the effect of organic solvents on aqueous solutions of plasma protein products, sufficient chloroform was added to aqueous solutions of Factor VIII to comprise 20 vol. % of the resultant mixture. The Factor VIII solutions were prepared by dissolving lyophilized commercial Factor VIII in water for injection to form solutions containing about 1 wt. % or about 6 wt. % of protein matter prior to addition of the organic liquid. The aqueous and organic liquids were cooled to about 10°C, then combined, and vortexed at 6-7°C. Samples were drawn at 30, 60 and 90 minutes and centrifuged, the organic and aqueous phases were separated, and the aqueous layer was assayed for Factor VIII activity and protein. The organic phase contained precipitate which could not successfully redissolve in water. The protein lost biological activity as shown in Table I compared to samples of the starting commercial Factor VIII solution which had been likewise agitated but not treated with chloroform.

## TABLE 1

### 1% Protein

| Sample | Protein mg/ml | % Yield | Factor VIII Units/ml | % Yield | Specific Activity Units/Mg |
|---|---|---|---|---|---|
| Control | 13.1 mg | – | 26.4 units | – | 2.02 |
| 30 min. | 6.5 mg | 49.7% | 20.0 units | 75.8% | 3.08 |
| 60 min. | 5.1 mg | 39.1% | 15.9 units | 60.2% | 3.12 |
| 90 min. | 5.06 mg | 38.6% | 15.3 units | 57.9% | 3.02 |

### 6% Protein

| Sample | Protein mg/ml | % Yield | Factor VIII Units/ml | % Yield | Specific Activity Units/Mg |
|---|---|---|---|---|---|
| Control | 64.3 mg | – | 32.4 units | – | 0.50 |
| 30 min. | 23.9 mg | 37.1% | 7.5 units | 23.1% | 0.31 |
| 60 min. | 21.9 mg | 34.1% | 4.1 units | 12.7% | 0.19 |
| 90 min. | 21.9 mg | 34.1% | 3.8 units | 11.7% | 0.17 |

-15-

The loss of activity is due to irreversible precipitation of proteins as well as the solvent's effect on active biological principle.

The precipitation of proteins can be demonstrated even with very diluted protein solution and using a water-immiscible solvent (chloroform). A lot of lyophilized commercial Factor VIII was reconstituted in water for injection to make a solution comprising 0.6% protein. This was further diluted with water for injection or citrate-glycine buffer to 0.4%, 0.2% and 0.1% protein. The protein solutions and chloroform were cooled to 10°C, then 1.2 ml of chloroform was added to 5 ml of each of the protein solutions and the samples were vortexed for 20 minutes. Each sample was mixed for 20-second intervals and then returned to the 10°C bath in order to maintain the temperature close to 10°C during the 20 minutes of mixing. The mixture was filtered and then the $OD_{280}$ was determined for chloroform-treated and untreated controls. The $OD_{280}$ corresponds directly to the amount of protein and thus the biological activity, in the solution.

| Protein Concentration | $CHCl_3$ | $OD_{280}$ | % Yield |
|---|---|---|---|
| 0.6% | No | 8.73 | |
| | Yes | 2.76 | 31.6% |
| 0.4% | No | 5.38 | |
| | Yes | 1.08 | 29% |
| 0.2% | No | 2.60 | |
| | Yes | 1.51 | 58% |
| 0.1% | No | 1.31 | |
| | Yes | 0.90 | 69% |

-16-

EXAMPLE 3

To demonstrate that organic solvent does not adversely affect the activity of dry plasma protein, batches of the same lyophilized commercial Factor VIII used in Examples 1 and 2 were slurried in chloroform at about 200 grams of dry material per ml of solvent. The solvent was filtered off after contact times of up to 8 hours.

Samples of this treated Factor VIII were then reconstituted with water for injection and tested in comparison to untreated commercial Factor VIII, and were found to have undergone no change in chemical and biological properties detectable to the limits of the analytical techniques employed, when tested as to reconstitution characteristics, biological activity, stability, gel electrophoresis, immunochemical identity, light absorbence, in vivo tolerance, in vivo recovery, and in vivo half-life.

Some samples of the treated Factor VIII were reconstituted to about 16 mg of protein per ml and then assayed for biological activity.

| Mode of $CHCl_3$ Treatment | Concentration on Reconstitution | Activity On Reconstitution |
|---|---|---|
| Control (not treated with solvent) | 15.8 mg/ml | 9.1 units/ml |
| 6 hrs at room temperature | 15.7 mg/ml | 9.5 units/ml |
| 6 hrs at 4°C | 15.6 mg/ml | 8.9 units/ml |
| 6 hrs twice at 4°C | 16 mg/ml | 9.3 units/ml |

-17-

EXAMPLE 4

A commercial Factor IX Complex preparation was lyophilized and slurried in chloroform at 200 mg of dry protein material per ml of solvent, and the solvent was filtered off after a contact time of 6 hours. The product was reconstituted into injectable solution form and found to have undergone no change in chemical or biological properties. The Non-Activated Plasma Thromboplastin Time (NAPTT) as described by H. S. Kingdon et al., Thrombos. Diathes. haemorrh. (Stuttg.), 1975, 33, 617-631 was performed for evaluation of potential thrombogenicity. The results, shown in Table II, demonstrate that solvent treatment has substantially improved the quality of the product.

### Table II

(Blank was with buffer instead of Factor IX)

Factor IX not treated with chloroform:
      1:10 dilutions  =   79% of blank
      1:100 dilutions =   95% of blank

Factor IX chloroform treated:
      1:10 dilutions  =   94% of blank
      1:100 dilutions = 100% of blank

-18-

WHAT IS CLAIMED IS:

1. A process for preventing the transmission of virus having structural lipids by an injectable plasma protein product, while retaining at least about 25% of the biological activity of the plasma protein product, comprising

(a) forming a slurry by mixing the plasma protein product in the dry state with a solvent which is capable of dissolving lipids and in which the plasma protein product is essentially insoluble.

(b) maintaining the slurry under conditions effective to extract said material into the organic solvent while lowering the biological activity of said product by no more than about 75%, and then

(c) removing the solvent in the liquid state from the plasma protein product, thereby providing the product in a dry, purified state.

2. The process of claim 1 wherein the virus is hepatitis B or hepatitis non-A-non-B viruses.

3. The process of claim 1 or 2 wherein the plasma protein product is of the type more than about 10% of which is precipitated from a 0.1 wt. % aqueous solution thereof at 10°C by mixing such aqueous solution with 20 vol. % of chloroform

4. The process of claim 3 wherein the plasma protein is Factor VIII, Factor IX, Factor IX complex, albumin, or Antithrombin III.

5. The process of any of claims 1 to 3 wherein the plasma protein product is Factor IX or Factor IX complex, and the thrombogenicity of said plasma protein product is reduced.

6. The process of claim 1 wherein the plasma protein product retains at least from 50% to 90% of its biological activity.

0112563

-19-

7. The process of any of claims 1 to 6 wherein the solvent that is removed in step (c) contains viable virus.

8. The process of claim 4 wherein the dry Factor VIII slurried in step (a) has been lyophilized from an aqueous solution thereof.

9. The process of any of claims 1 to 8 wherein the slurry in step (b) is maintained at about 2°C to about 30°C for up to about 8 hours.

10. The process of any of claims 1 to 9 wherein the organic solvent is a liquid at 25°C and atmospheric pressure and is of benzene, furans, alkanols, ketones, ethers, alkyl-substituted benzene, alkanes, and cyclo-alkanes, any of which may have up to 8 carbon atoms; and methane or ethane having 2 or more halogen substituents selected from fluorine or chlorine; and mono- and biphasic mixtures thereof.

11. The process of any of claims 1 to 10 which include formulating the dry, purified product provided in step (c) into an injectable therapeutic composition by dissolving said product in a pharmaceutically-acceptable liquid carrier.

12. The process of any of claims 1 to 11 wherein pyrogens are removed from the plasma protein product.

13. A process for reducing the thrombogenicity of an injectable plasma protein product selected from the group consisting of Factor IX and Factor IX Complex while retaining at least about 25% of the biological activity of said product, comprising

(a) forming a slurry by mixing the plasma protein product in the dry state with a solvent which is capable of dissolving lipids and in which the plasma protein product is essentially insoluble,

-20-

(b)   maintaining the slurry under conditions effective to reduce the thrombogenicity of said product while lowering the biological activity of said product by no more than about 75%, and then

(c)   removing the solvent in the liquid state from the plasma protein product, thereby providing said product in a dry, purified state having lowered thrombogenicity.